# EUROPEAN PATENT APPLICATION

(11) **EP 1 903 118 A2**
(43) Date of publication of application: **26.03.2008**
(21) Application number: 07116743.1
(22) Date of filing: 19.09.2007
(51) Int. Cl.: C12Q 1/68

(54) **Method for confirming cytosine conversion treatment and nucleic acid molecule used therefor**

(30) Priority: 21.09.2006 JP 2006255808
(71) Applicant: SYSMEX CORPORATION, Kobe-shi, Hyogo 651-0073 (JP)
(72) Inventor: Masahiro, Kajita, Kobe-shi Hyogo 651-0073 (JP); Noriaki, Yamamoto, Kobe-shi Hyogo 651-0073 (JP); Ayako, Sakai, Kobe-shi Hyogo 651-0073 (JP); Hideki, Ishihara, Kobe-shi Hyogo 651-0073 (JP)
(74) Representative: De Clercq, Ann G. Y.

(57) **Abstract**

There is provided a method for confirming conversion treatment of non-methylated cytosine, including a step of mixing a DNA taken from a living body, with a nucleic acid for accuracy control having a sequence A which is not contained in a genome of the living body and contains cytosine, a step of converting non-methylated cytosine in the mixture into a base other than cytosine, a step of detecting the sequence A and/or a sequence A' which has a sequence obtained by converting cytosine in the sequence A into the base other than cytosine, and a step of determining whether the conversion step has been properly performed or not based on the result of the detection step.

SEQUENCE LISTING

<110>  SYSMEX Corporation

<120>  Method for confirming conversion treatment and nucleic acid molecule used therefor

<130>  SYSM-014-EP

<150>  JP 2006-255808
<151>  2006-09-21

<160>  20

<170>  PatentIn version 3.1

<210>  1
<211>  25
<212>  DNA
<213>  Artificial

<220>
<223>  Designed BS sequence

<400>  1
gggccggcaa gccacgaacc caccc                                          25


<210>  2
<211>  25
<212>  DNA
<213>  Artificial

<220>
<223>  Designed BS sequence

<400>  2
ccccgagccg cacgcgccac cgagg                                          25


<210>  3
<211>  25
<212>  DNA
<213>  Artificial

<220>
<223>  Designed Uni sequence

<400>  3
gtttatgttt ttggggtgtt ggaag                                          25


<210>  4
<211>  24
<212>  DNA
<213>  Artificial

<220>
<223>  Designed Uni sequence

<400>  4
gaggggataa gaatttgaga gagg                                           24


<210>  5
<211>  21
<212>  DNA
<213>  Artificial

<220>
<223>  Designed sequence for biding TaqMan probe

<400>  5
tgggtgggtt gtgaggtggt g                                              21


<210>  6
<211>  50

<212>  DNA
<213>  Artificial

<220>
<223>  Designed sequence for synthesizing quality control nucleic acid

<400>  6
gtttatgttt ttggggtgtt ggaaggggcc ggcaagccac gaacccaccc          50


<210>  7
<211>  51
<212>  DNA
<213>  Artificial

<220>
<223>  Designed sequence for synthesizing quality control nucleic acid

<400>  7
cctctctcaa attcttatcc cctcatcctc ggtggcgcgt gcggctcggg g          51


<210>  8
<211>  73
<212>  DNA
<213>  Artificial

<220>
<223>  Designed sequence for synthesizing quality control nucleic acid

<400>  8
gggccggcaa gccacgaacc caccctgtgg gtgggttgtg aggtggtgcc ccgagccgca     60

cgcgccaccg agg                                                        73


<210>  9
<211>  124
<212>  DNA
<213>  Artificial

<220>
<223>  Quality control nucleic acid

<400>  9
gtttatgttt ttggggtgtt ggaaggggcc ggcaagccac gaacccaccc tgtgggtggg     60

ttgtgaggtg gtgccccgag ccgcacgcgc caccgaggat gaggggataa gaatttgaga    120

gagg                                                                 124


<210>  10
<211>  25
<212>  DNA
<213>  Artificial

<220>
<223>  Designed BS-F primer

<400>  10
gggttggtaa gttatgaatt tattt                                           25


<210>  11
<211>  25
<212>  DNA
<213>  Artificial

<220>
<223>  Designed BS-R primer

<400>  11
cctcaataac acatacaact caaaa                                           25


<210>  12

<211>  16
<212>  DNA
<213>  Artificial

<220>
<223>  Designed nonBS-F primer

<400>  12
aagccacgaa cccacc                                                        16


<210>  13
<211>  15
<212>  DNA
<213>  Artificial

<220>
<223>  Designed nonBS-R primer

<400>  13
tcatcctcgg tggcg                                                         15


<210>  14
<211>  22
<212>  DNA
<213>  Artificial

<220>
<223>  Desinged Ecad-F primer

<400>  14
ttaggttaga gggttatcgc gt                                                 22


<210>  15
<211>  23
<212>  DNA
<213>  Artificial

<220>
<223>  Designed Ecad-R primer

<400>  15
taactaaaaa ttcacctacc gac                                                23


<210>  16
<211>  25
<212>  DNA
<213>  Artificial

<220>
<223>  Designed ER-F primer

<400>  16
ttttgggatt gtatttgttt tcgtc                                              25


<210>  17
<211>  24
<212>  DNA
<213>  Artificial

<220>
<223>  Designed ER-R primer

<400>  17
aacaaaatac aaaccgtatc cccg                                               24


<210>  18
<211>  23
<212>  DNA
<213>  Artificial

<220>

<223>  Designed Uni-F primer

<400>  18
gtttatgttt ttgggtgtt gga                                                23


<210>  19
<211>  24
<212>  DNA
<213>  Artificial

<220>
<223>  Designed Uni-R primer

<400>  19
cctctctcaa attcttatcc cctc                                              24


<210>  20
<211>  19
<212>  DNA
<213>  Artificial

<220>
<223>  Designed probe

<400>  20
tgggtgggtt gtgaggtgg                                                    19

## Description

### FIELD OF THE INVENTION

The present invention relates to a method for confirming whether conversion treatment, which is conducted upon detection of methylation of cytosine in a predetermined region of a DNA, was properly done or not, and a nucleic acid molecule and a reagent kit used therefor.

### BACKGROUND

In a chromosomal DNA of a higher eukaryote, a 5-position of C (cytosine) of bases constituting a DNA undergoes methylation modification in some cases. This methylation of a DNA in a higher eukaryote functions as a mechanism for suppressing expression of genetic information. In a genomic DNA, a region containing a large amount of CpG (CpG island, CG island) is present in a promoter region of a gene in many cases. For this reason, when a CpG island has been methylated, a transcription factor can not bind to a promoter, and transcription of a gene is suppressed. When a CpG island has not undergone methylation, it becomes possible for a transcription factor to bind to a promoter region, resulting in the state where a gene can be transcribed. Controlling of gene expression by such methylation of a DNA plays an important role in various physiological or pathological phenomena such as early embryo development, tissue-specific gene expression, gene stenciling or inactivation of an X chromosome which is a characteristic phenomenon in a mammal, stabilization of a chromosome, and timing of DNA replication. Further, in recent years, it has been revealed that this DNA methylation is strongly involved in cancer and other diseases.

Cytosine which has not been methylated in a DNA (non-methylated cytosine) is converted into another base by conversion treatment (treatment for converting cytosine into another base (e.g. treatment of contacting a reagent of converting a base such as bisulfite (hereinafter, referred to as a converting agent) with a DNA)). However, cytosine which has been methylated (methylated cytosine) is not converted into another base even when conversion-treated. Utilizing this, a DNA in a specimen which is to be detected is conversion-treated, and whether cytosine has been converted into another base or not is detected by a methylation-specific PCR method (MS-PCR, James G. HERMAN et al., Methylation-specific PCR: A novel PCR assay FOR methylation status of CpG islands, Proc. Natl. Acad. Sci. USA, Vol. 93, pp. 9821-9826, September 1996) or the like. Thereby, whether a specified region of the DNA has been methylated or not can be detected (methylation detection) (US6017704).

However, if conversion treatment is not properly performed, an erroneous result may be generated upon methylation detection. For this reason, upon detection of methylation in a specimen, it is preferable to confirm whether conversion treatment has been properly performed or not.

As a control for confirming that conversion treatment has been properly performed, a control DNA derived from the human genome known to contain non-methylated cytosine (e.g. CpGenome TM Universal Unmethylated DNA (CHEMICON) or the like) can be used. In addition, as a control for confirming that methylated cytosine is not converted even when conversion-treated, a control DNA derived from the human genome containing methylated cytosine (e.g. CpGenome TM Universal Methylated DNA (CHEMICON) or the like) can be used.

By performing conversion treatment and methylation detection of the aforementioned controls accompanying with conversion treatment and methylation detection of a DNA contained in a specimen, whether the specimen has been properly conversion-treated or not can be presumed.

### SUMMARY

The scope of the present invention is defined solely by the appended claims, and is not affected to any degree by the statements within this summary.

Since the aforementioned control DNAs are derived from the human genome, when each of them is mixed with a specimen taken from a human, and conversion treatment and methylation detection are performed in the same container, which of methylation derived from a specimen or a control DNA is detected can not be determined upon methylation detection after conversion treatment. Therefore, when the aforementioned control DNA having a sequence derived from the human genome is used, it is necessary to perform conversion treatment in separate containers. However, conversion treatment can not be performed under the same condition in separate containers. For this reason, whether a specimen has been properly conversion-treated or not can not be confirmed with high reliance based on the result of methylation detection of the aforementioned control DNA.

The present invention was made in view of the aforementioned circumstances, and an object thereof is to provide a method for confirming conversion treatment, which can determine whether conversion treatment has been properly performed or not, with higher reliance than the conventional one, and a reagent kit for confirmation.

The present invention provides a method for confirming whether conversion treatment, which converts non-methylated cytosine of a DNA taken from a living body into a base other than cytosine, was properly performed or not, comprising a step of: mixing the DNA taken from the living body, with a nucleic acid for accuracy control having a sequence A which is not contained in a genome of the living body and contains cytosine; a step of converting non-methylated cytosine in the mixture, which is obtained in the above step, into a base other than cytosine; a step of detecting the sequence A or a sequence A', wherein sequence A' has a sequence obtained by converting cytosine in the sequence A into the base other than cytosine; and a step of determining whether the conversion step has been properly performed or not based on the result of the above detection step.

Also, the present invention provides a nucleic acid molecule comprising any of the following sequences:
(a) a nucleotide sequence of SEQ ID No. 1;
(b) a nucleotide sequence in which at least one nucleotide is substituted, deleted, inserted or added in the nucleotide sequence of SEQ ID No. 1, and which is not contained in the human genome;
(c) a nucleotide sequence of SEQ ID No. 2; and
(d) a nucleotide sequence in which at least one nucleotide is substituted, deleted, inserted or added in the nucleotide sequence of SEQ ID No. 2, and which is not contained in the human genome.

Further, the present invention provides a reagent kit for confirming conversion treatment, comprising a nucleic acid for accuracy control having a sequence A which is not contained in a genome of the living body and contains cytosine, and at least one polynucleotide selected from a polynucleotide being capable of hybridizing with the sequence A, and a polynucleotide being capable of hybridizing with a sequence A' having a sequence obtained by converting cytosine in the sequence A into the base other than cytosine.

According to the present invention, whether treatment of converting non-methylated cytosine into a base other than cytosine was properly performed on a DNA or not can be confirmed with high reliance.

### BRIEF DESCRIPTION OF THE DRAWINGS

Fig. 1 is a schematic view showing a method for confirming conversion treatment in accordance with a conventional method, and a method for confirming conversion treatment in accordance with the present embodiment;
Fig. 2 (a) is a schematic view of a nucleic acid for accuracy control having a first Uni sequence, a first BS sequence, a sequence for a probe, a second BS sequence and a second Uni sequence in this order from a 5'-end side, and Fig. 2(b) is a schematic view showing with which region of a nucleic acid for accuracy control, the nucleic acid for accuracy control, a primer and a probe hybridize;
Fig. 3 is a photograph of an agarose gel showing a result of detection of methylation of the nucleic aid for accuracy control when conversion treatment is performed;
Fig. 4 is a photograph of an agarose gel showing a result of detection of methylation of the nucleic aid for accuracy control when conversion treatment is not performed;
Fig. 5 is a photograph of an agarose gel showing a result of detection of methylation of Ecad and ER when conversion treatment is performed; and
Fig. 6 is a photograph of an agarose gel showing a result of detection of methylation of Ecad and ER when conversion treatment is not performed.

### DETAILED DESCRIPTION OF THE EMBODIMENT

A confirmation method which is one embodiment of the present invention comprises a step of mixing a specimen containing a DNA for which methylation is detected, and a nucleic acid for accuracy control (mixing step), a step of converting non-methylated cytosine in the mixture obtained in the mixing step into a base other than cytosine (conversion step), a step of detecting whether cytosine of the nucleic acid for accuracy control contained in the mixture was converted into the base other than cytosine (detection step), and a step of determining whether the conversion treatment in the conversion step was properly performed or not based on the detection result obtained in the detection step (determination step).

A specimen is not particularly limited, as far as it contains a genomic DNA of a living body. For example, blood, serum, lymph liquid, urine, mamilla secreta, and a part of a tissue taken by operation or biopsy can be used. Since a genomic DNA is present in a cell, it is preferable to release a genomic DNA from a cell into a solution before mixing with a nucleic acid for accuracy control. For example, a genomic DNA can be extracted and purified with a commercially available kit. Alternatively, by mixing a buffer containing a surfactant which solubilizes a cell, with a specimen, and performing physical treatment (stirring, homogenization, ultrasound treatment or the like), a genomic DNA in a cell can be released into a solution. In this case, after physical treatment, a cell piece is precipitated by centrifugation, and a supernatant containing a genomic DNA can be subjected to later conversion treatment and methylation analysis. By using such a buffer, the extraction and purification of a genomic DNA with the aforementioned kit become unnecessary, and a sample for detection can be obtained in a short time and simply only by a simple centrifugation operation. Herein, the surfactant used is not particularly limited, as far as it can solubilize a cell, but sodium cholate or sodium dodecylsulfate (SDS) can be suitably used.

The nucleic acid for accuracy control may be a DNA or an RNA, and may be single-stranded or double-stranded and, from the viewpoint of stability, a double-stranded DNA is most preferable.

The nucleic acid for accuracy control has a sequence which is not contained in a genome in a specimen. This sequence is a sequence which contains at least one base of cytosine, and is to be conversion-treated (hereinafter, this sequence is referred to as a BS sequence). For example, when a specimen is taken from a human, the specimen contains the human genome. Therefore, the BS sequence may be an artificially designed sequence, or a sequence contained in a genome of other organism species, as far as it is a sequence which is not contained in the human genome.

For example, a molecule composed of a nucleic acid represented by the following sequence (nucleic acid molecule) can be exemplified.
5'-gggccggcaagccacgaacccaccc-3' SEQ ID NO. 1
5'-ccccgagccgcacgcgccaccgagg-3' SEQ ID No. 2

These sequences are sequences which are not contained in the human genome, and the nucleic acid for accuracy control may contain either one of these sequences, or both of them. A sequence in which one or more nucleotide(s) is/are mutated (substituted, deleted, inserted, added or the like) in these sequences can be used as a BS sequence, as far as it is a sequence which is not contained in the human genome and contains one or more base(s) of cytosine.

When methylation is detected with a DNA chip, it is enough that the nucleic acid for accuracy control contains either one polynucleotide of SEQ ID No. 1 and SEQ ID No. 2. In this case, a substrate on which a polynucleotide hybridizing with this polynucleotide is immobilized can be used as a DNA chip. In addition, when methylation is detected by MS-PCR, it is preferable that the nucleic acid for accuracy control contains both of the above sequences. In this case, a polynucleotide which hybridizes with a chain complementary with one of the above sequences, and a polynucleotide hybridizing with other sequence can be used as a primer set.

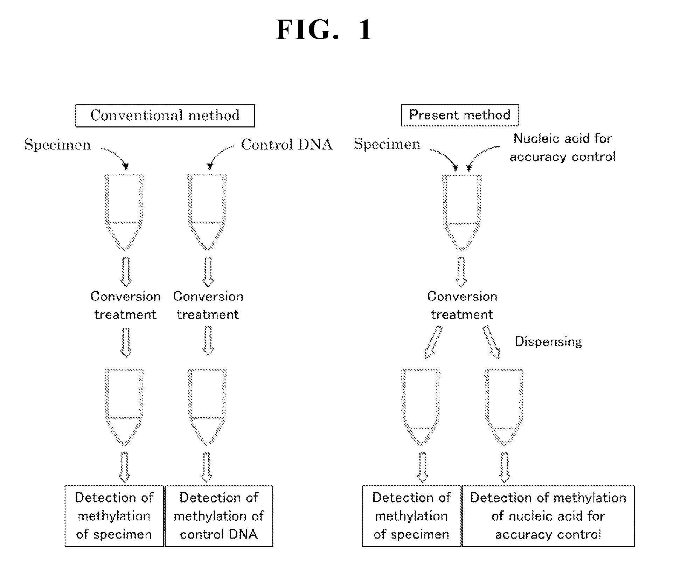

Since the BS sequence is a sequence which is not contained in a genome in a specimen, a polynucleotide which specifically hybridizes with the BS sequence does not substantially hybridize with a genome in a specimen, and a polynucleotide which specifically hybridizes with a genome in a specimen does not substantially hybridizes with the BS sequence. For this reason, depending on use of either of a polynucleotide which is specific for a genome and a polynucleotide which is specific for the BS sequence, which methylation is detected can be discriminated even in a solution in which both of the nucleic acid for accuracy control and a specimen are present. Therefore, it becomes possible to perform conversion treatment on a specimen and conversion treatment on the nucleic acid for accuracy control simultaneously in the same container (see Fig. 1). Fig. 1 is a schematic view showing a method for confirming conversion treatment in accordance with a conventional method and a method for confirming conversion treatment in accordance with the present embodiment. In the conventional method, a container containing a specimen and a container containing a control DNA are separately prepared, conversion treatment is performed in each container and, after conversion treatment, detection of methylation of the specimen or the control DNA is performed on each container. In the method of the present embodiment, a specimen and a nucleic acid for accuracy control are mixed and, after conversion treatment, a mixture after conversion treatment is dispensed in order to perform detection of methylation of the specimen and detection of methylation of the nucleic acid for accuracy control.

When a TaqMan (registered trade mark of Roche Diagnostic) PCR method (Heid et al., 1996, Genome Research, Vol.6 6 (10), 986-994) is used, it is possible to perform detection of methylation of a specimen and detection of methylation of a nucleic acid for accuracy control in the same container without dispensing a mixture, after conversion treatment (see Panda et al., 2002, Cell, Vol. 109, 307-320). In this case, a primer set (forward primer and reverse primer) hybridizing with a specimen, a primer set hybridizing with a nucleic acid for accuracy control, a TaqMan probe A hybridizing with a specimen, and a TaqMan probe B hybridizing with a nucleic acid for accuracy control are added to a mixture after conversion treatment. Thereupon, for example, when the TaqMan probe A and the TaqMan probe B are labeled with fluorescent substances emitting fluorescence lights having different wavelengths, respectively, detection of methylation of a specimen and detection of methylation of a nucleic acid for accuracy control can be performed depending on a wavelength of light emitted from the mixture when excitation light is irradiated, without dispensing the mixture after conversion treatment. Alternatively, it is also possible to combine a TaqMan PCR method and a SYBR Green method. For example, when detection of methylation of a specimen is performed by a SYBR Green method, and detection of methylation of a nucleic acid for accuracy control is performed by the TaqMan PCR method, detection of methylation of the specimen and detection of methylation of the nucleic acid for accuracy control can be performed in the same container, without dispensing a mixture after conversion treatment.

By performing conversion treatment on a specimen and conversion treatment on a nucleic acid for accuracy control under the entirely same condition, whether conversion treatment on a specimen was proper or not can be confirmed with higher reliance based on the result of detection of methylation of a nucleic acid for accuracy control. In addition, by performing these conversion treatments in the same container, operation can be conducted simpler than in the case the conversion treatment is performed in separated containers, and a consumed amount of a reagent can be reduced.

A mixture obtained by mixing a specimen and a nucleic acid for accuracy control is subjected to conversion treatment (conversion step). The conversion treatment is treatment of converting non-methylated cytosine into a base other than cytosine (i.e. uracil, thymine, adenine or guanine). In the conversion step, preferably, a converting agent of converting non-methylated cytosine into a base other than cytosine is added to a mixture. As such converting agent, bisulfite is preferably used. As the bisulfite, a sodium salt, a potassium salt, a calcium salt, and a magnesium salt of bisulfite can be used. When a DNA is treated using any one or more of them, non-methylated cytosine in a DNA is converted into uracil by deamination. On the other hand, the aforementioned converting agent does not act on methylated cytosine in a DNA, and conversion into another base does not occur. It is preferable that this converting agent is dissolved in a suitable solvent such as purified water or a buffer, and is used as a solution. When bisulfite is used as the converting agent, an amount of addition to the mixture is not limited, as far as it is such an extent that non-methylated cytosine of a DNA in a sample can be sufficiently converted. For example, when 6 mmol of sodium bisulfite is added to a mixture, conversion of non-methylated cytosine into uracil can be performed by incubation at 50 to 80°C for 10 to 30 minutes. Alternatively, when bisulfite is used at a low concentration (e.g. 520 µl of a 3M sodium bisulfite solution), conversion may be performed at a temperature for a time to such an extent that non-methylated cytosine can be sufficiently converted. The converting agent is not limited to bisulfite, and any converting agent can be used, as far as it does not convert methylated cytosine and can selectively convert non-methylated cytosine into another base.

By such conversion treatment, a DNA sequences is different between the case where cytosine at a particular region of a DNA and in a nucleic acid for accuracy control in a specimen is methylated, and the case where the cytosine is not methylated. By analyzing this difference in a DNA sequence using the aforementioned MS-PCR, DNA chip or DNA sequencer, the presence or absence of methylation of a DNA in a specimen, and of a nucleic acid for accuracy control can be detected.

When MS-PCR or a DNA chip is used for analyzing methylation of a nucleic acid for accuracy control, whether cytosine was converted or not can be detected by using a polynucleotide hybridizing with the BS sequence when non-methylated cytosine was converted into another base (hereinafter, referred to as polynucleotide U), and a polynucleotide hybridizing with the BS sequence when non-methylated cytosine was not converted (hereinafter, referred to as polynucleotide M). These polynucleotides can be hybridized under the condition stringent to the BS sequence. In the case of PCR, the stringency in these nucleic acid hybridization reactions depends mainly on a temperature and a salt concentration in a PCR reaction solution, and the stringency becomes higher (hybridization becomes difficult) at a higher temperature and a lower salt concentration (see J. Sambrook et al., Molecular Cloning, A Laboratory Manual, 2nd ed., Cold Spring Harbor Laboratory (1989)). In addition, when the DNA chip is used, hybridization can be performed in the presence of formamide or the like. As a specific example of the stringent condition, "in a solution containing 50% formamide, 5 × SSC (150 mM NaCl, 15 mM sodium citrate), 50 mM sodium phosphate, pH 7.6, 5 × Denhardt solution, 10% dextran sulfate, and 20 µg/ml nucleic acid, a hybridization temperature of 42°C" can be exemplified, being not limiting. In the present specification, "hybridizing" means hybridization under the stringent condition.

When methylation is detected by MS-PCR, it is preferable that the nucleic acid for accuracy control has a first BS sequence and a second BS sequence. In this case, two kinds of polynucleotides U (referred to as a primer set U: a forward primer U hybridizing with a first BS sequence when cytosine was converted into another base, and a reverse primer U hybridizing with a second BS sequence when cytosine was converted into another base), and two kinds of polynucleotides M (referred to as a primer set M: a forward primer M hybridizing with a first BS sequence when cytosine was not converted, and a reverse primer M hybridizing with a second BS sequence when cytosine was not converted) can be used.

When methylation is detected by MS-PCR, it is preferable that a mixture after conversion treatment is dispensed into at least four containers. Among these containers, two (referred to as a container A and a container B) are used for detecting methylation of a DNA in a specimen, and remaining two (referred to as a container C and a container D) can be used for detecting methylation of a nucleic acid for accuracy control.

In the container A, methylation is detected using a primer hybridizing with a sequence when cytosine at a particular region of a specimen DNA was converted into a base other than cytosine by conversion treatment. In the container B, methylation is detected using a primer hybridizing with a sequence when cytosine at a particular region of a specimen DNA was not converted into another base. When amplification of a nucleic acid is recognized in the container A, and amplification of a nucleic acid was not recognized in the container B, it is presumed that cytosine at a particular region was non-methylated cytosine. When amplification of a nucleic acid was recognized in the container B, and amplification of a nucleic acid was not recognized in the container A, it is presumed that cytosine at a particular region was methylated cytosine.

On the other hand, in the container C, methylation is detected using a primer hybridizing with a BS sequence when cytosine of a BS sequence of a nucleic acid for accuracy control was converted into a base other than cytosine by conversion treatment. In the container D, methylation is detected using a primer hybridizing with a BS sequence when cytosine of a nucleic acid for accuracy control was not converted.

When cytosine contained in a BS sequence of a nucleic acid for accuracy control is non-methylated cytosine, if conversion treatment is proper, cytosine in the BS sequence is converted into a base other than cytosine. For this reason, when amplification for a nucleic acid for accuracy control was detected in the container C, and amplification of a nucleic acid for accuracy control was not detected (or an amplification efficiency was remarkably bad) in the container D, it is seen that non-methylated cytosine in the BS sequence was properly converted into a base other than cytosine. Based on this result, it can be determined that non-methylated cytosine of a DNA in a specimen was converted into a base other than cytosine, and conversion treatment was properly performed. Therefore, it can be thought that the detection result obtained in the containers A and B has high reliance.

On the other hand, when amplification of a nucleic acid for accuracy control was not detected (or an amplification efficiency was remarkably bad) in the container C, and amplification of a nucleic acid for accuracy control was detected in the container D, it is seen that non-methylated cytosine in the BS sequence was conversion-treated, but was not converted into a base other than cytosine. Based on this result, it can be determined that non-methylated cytosine of a DNA in a specimen was not converted into a base other than cytosine, and conversion treatment was not properly performed. Therefore, it can be thought that the detection result obtained in the containers A and B has low reliance and, in response to this, a countermeasure such as re-experiment can be taken.

When cytosine contained in a BS sequence of a nucleic acid for accuracy control is methylated cytosine, if conversion treatment was proper, cytosine in the BS sequence is not converted into a base other than cytosine. For this reason, when amplification of a nucleic acid for accuracy control was not detected (or an amplification efficiency was remarkably bad) in the container C, and amplification of a nucleic acid for accuracy control was detected in the container D, it is seen that methylated cytosine in the BS sequence was not converted by the conversion treatment. Therefore, it can be determined that methylated cytosine of a DNA in a specimen was also not converted into another base, and conversion treatment was properly performed.

On the other hand, when amplification of the nucleic acid for accuracy control was detected in the container C, and amplification of the nucleic acid for accuracy control was not detected (or an amplification efficiency was remarkably bad) in the container D, it is seen that methylated cytosine in the BS sequence had been converted into another base by conversion treatment. Therefore, it can be determined that methylated cytosine in a specimen was also converted into another base, and conversion treatment was not properly performed.

That is, by detecting, after conversion treatment, a BS sequence contained in the nucleic acid for accuracy control and/or a BS sequence in which cytosine was converted into another sequence, whether conversion treatment is proper or not can be determined.

Upon detection of methylation of a nucleic acid for accuracy control or a specimen by MS-PCR or a DNA chip, it is preferable to determine the presence or absence of methylation by comparing the detection result with a predetermined threshold. For example, when MS-PCR is used, a threshold can be set as follows.

First, using a plurality of specimens, methylation at a particular region of a genome is detected by quantitative real time PCR. Herein, the number of cycles from a reaction solution to reaching a signal such as a fluorescent intensity of a predetermined value in real time PCR is designated as a detection cycle number. A detection cycle number upon use of a primer set U of a specimen determined to be methylated (methylated specimen) and a detection cycle number upon use of a primer set U of a specimen determined to be not methylated (non-methylated specimen) are compared, a value by which a methylated specimen and a non-methylated specimen can be separated at a highest probability can be used as a threshold. When the detection cycle number is not less than this threshold, the specimen is determined to be methylated and, when the number is less than this threshold, the specimen is determined to be not methylated.

Furthermore, a detection cycle number upon use of a primer set M of a methylated specimen and a detection cycle number upon use of a primer set M of a non-methylated specimen are compared, a value by which a methylated specimen and a non-methylated specimen can be separated at a highest probability may be used as a threshold. When the detection cycle number is not less than this threshold, the specimen is determined to be not methylated and, when the number is less than this threshold, the specimen is determined to be methylated.

The presence or absence of methylation of a specimen may be determined using both of a threshold upon use of a primer set U and a threshold upon use of a primer set M, or using either one of them.

It is preferable to purify a DNA after conversion treatment and before methylation detection. For purifying a DNA, a commercially available purification kit (e.g. QIAquik (trade name) manufactured by Qiagen, or the like) can be used.

Upon detection of methylation of a DNA of a specimen, it is preferable to calculate to what an extent a DNA was able to be recovered (hereinafter, also referred to as a recovery rate) as compared with at the mixing step. When DNA purification or conversion treatment is performed, a nucleic acid is lost, and a DNA amount of a mixture before methylation detection is decreased as compared with at the mixing step. The recovery rate can be useful information in setting a threshold or determining methylation.

When the recovery rate is low, it is necessary to strictly set a threshold for determining whether methylated or not, but when the recovery rate is high, a threshold can be set flexibly.

For example, the case where methylation at a promoter region of a gene is detected using quantitative real time PCR will be explained below. When using a predetermined detection cycle number as a threshold, amplification is confirmed (or amplification is not confirmed) at a detection cycle number equal to or more than a threshold in a primer set M, and amplification is confirmed at a detection cycle number less than a threshold in a primer set U, it can be determined that cytosine in the promoter region is not methylated.

On the other hand, when amplification is confirmed at a detection cycle number less than a threshold in a primer set M, and amplification is confirmed (or amplification is not confirmed) at a detection cycle number equal to or more than a threshold in a primer set U, it can be determined that a promoter region is methylated.

In such case, when the recovery rate is low, since a slight variation in the recovery rate greatly influences on a threshold, it is necessary to strictly set a threshold. For example, when a primer set M is added to a specimen for which the presence or absence of methylation is unknown, methylation is detected and, as a result, a detection cycle number is less than a threshold, it is difficult to determine whether a detection cycle number is less than a threshold due to non-methylation, or a detection cycle number is less than a threshold due to the low recovery rate (a small amount of a DNA which is to be a template of PCR). However, when the recovery rate is high, since a small variation in the recovery rate slightly influences on a threshold, a threshold can be set flexibly.

From the foregoing, it is useful to increase a recovery rate of a DNA in methylation detection and, thereupon, the technique of measuring a recovery rate becomes important.

Measurement of the recovery rate will be explained below.

In order to determine to what an extent a DNA in a specimen is decreased, in addition to the BS sequence, a nucleic acid for accuracy control further having a sequence which does not contain non-methylated cytosine, and is not contained in a genome in a specimen (hereinafter, referred to as a Uni sequence) can be used. It is preferable that the Uni sequence does not contain not only non-methylated cytosine but also methylated cytosine. The recovery rate can be measured using quantitative real time PCR or a DNA chip.

When an amount (mass, copy number or the like) of a nucleic acid for accuracy control to be mixed with a specimen is measured in advance and, after conversion treatment or DNA purification, an amount of the nucleic acid for accuracy control is quantitated using a polynucleotide being capable of hybridizing with the Uni sequence, the recovery rate of the nucleic acid for accuracy control can be calculated. Based on this value, to what an extent a genomic DNA of a specimen was decreased can be presumed. For example, when a specimen and 100 nucleic acids for accuracy control are mixed (mixing step) and, after conversion treatment and DNA purification, the result that 10 nucleic acids for accuracy control remain is derived by quantitative real time PCR, the recovery rate is 10%. Therefore, it can be presumed that the number of genomic DNA contained in a specimen also becomes 1/10 via conversion treatment and DNA purification as compared with at the mixing step.

As the Uni sequence, nucleic acid molecules represented by the following sequences can be exemplified.
5'-gtttatgtttttggggtgttggaag-3' (SEQ ID No. 3)
5'-gaggggataagaatttgagagagg-3' (SEQ ID No. 4)

These sequences are sequences not contained in the human genome, and the nucleic acid for accuracy control may contain either one of these sequences, or may contain both of them. A sequence in which one or more nucleotide(s) is/are mutated (substituted, deleted, inserted, added or the like) in these sequences can be used as the Uni sequence, as far as it is a sequence not contained in the human genome and not containing non-methylated cytosine.

When the nucleic acid for accuracy control is quantitated with a DNA chip, it is sufficient that either one of the polynucleotides is contained. In this case, a substrate on which a polynucleotide hybridizing with the above sequence is immobilized can be used as a DNA chip.

In addition, when the nucleic acid for accuracy control is quantitated by quantitative real time PCR, it is preferable that both of the above sequences are contained as the first Uni sequence and the second Uni sequence, the first Uni sequence is situated at a 5' -end of the nucleic acid for accuracy control, and the second Uni sequence is situated at a 3'-end of the nucleic acid for accuracy control. Thereby, when quantitative real time PCR is performed using, as a primer set, a polynucleotide hybridizing with the first Uni sequence, and a polynucleotide hybridizing with the second Uni sequence, a full length of the nucleic acid for a precision can be amplified, and a mass of the nucleic acid for accuracy control can be quantitated.

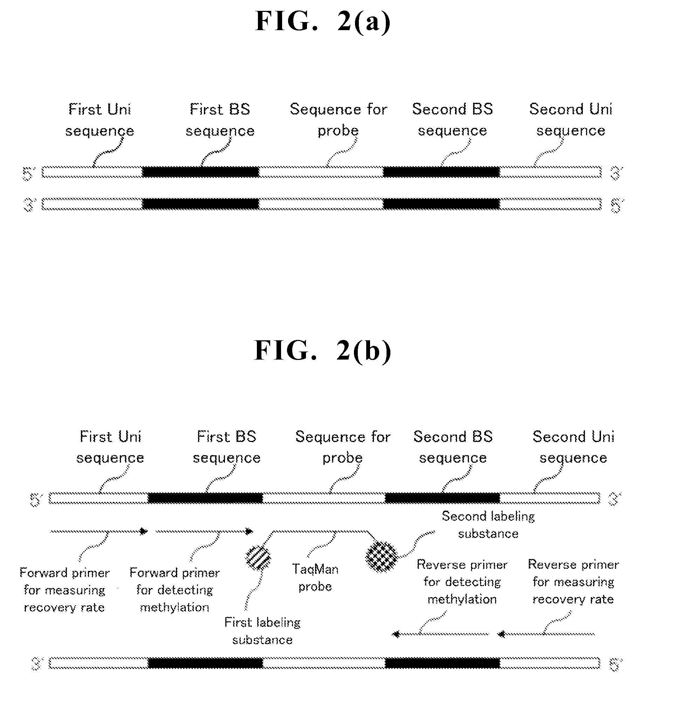

As the quantitative real time PCR, the known methods (e.g. a SYBR Green method, a TaqMan PCR method or the like) can be used. When the TaqMan PCR method is used, as shown in Fig. 2(a), the nucleic acid for accuracy control has a sequence (sequence for a probe) with which a TaqMan probe hybridizes, between the first Uni sequence and the second Uni sequence.

As the sequence for a probe, the following sequence can be exemplified.
5'-tgggtgggttgtgaggtggtg-3' (SEQ ID No. 5)

The sequence for a probe is not particularly limited in its sequence, as far as the TaqMan probe hybridizes therewith, and a sequence in which one or more nucleotide(s) is/are mutated (substituted, deleted, inserted, added or the like) in the above sequence for a probe can be used as a sequence for a probe.

As the TaqMan probe, a polynucleotide hybridizing with the above sequence, or a polynucleotide hybridizing with a strand complementary with the above sequence can be used. A first labeling substance is bound to one of a 5'-end and a 3'-end of the TaqMan probe, and a second labeling substance is bound to the other end. It is preferable that the first labeling substance and the second labeling substance are a substance which can emit fluorescent light by irradiating light having a predetermined wavelength (fluorescent substance). When the first labeling substance and the second labeling substance are situated at a distance less than a predetermined distance, the first labeling substance emits fluorescent light with the light having a predetermined wavelength, and fluorescent light of the second labeling substance is quenched by this fluorescent light. In addition, when they are isolated at a distance equal to or more than the predetermined distance, since fluorescent light of the first labeling substance does not reach the second labeling substance, both or one of the first labeling substance and the second labeling substance in a reaction solution emit(s) fluorescent light by irradiating the reaction solution with light having a predetermined wavelength.

A combination of the first labeling substance and the second labeling substance is not particularly limited as far as they have the aforementioned properties. Examples of the first labeling substance include 6-carboxyfluorescein (FAM), tetrachloro-6-carboxyfluorescein (TET), 2,7-dimethoxy-4,5-dichloro-6-carboxyfluorescein (JOE), hexochloro-6-carboxyfluorescein (HEX), VIC and the like, and examples of the second labeling substance include 6-carboxy-tetramethyl-rhodamine (TAMRA) and the like.

In order that the second labeling substance quenches fluorescent light emitted by the first labeling substance, it is necessary that these substances are within a predetermined distance. This predetermined distance is different depending on a kind of the labeling substance, and is preferably not more than about 100Å. Since this distance corresponds to a polynucleotide of about 30 mer, it is preferable that a length of a probe is within 30 mer. In addition, in order to maintain specificity, this probe is not less than 10 mer, preferably not less than 12 mer, more preferably not less than 15 mer in length. It is not necessary that a sequence of the TaqMan probe is completely complementary with the sequence for a probe (or a strand complementary with the sequence for a probe), and may have complementarity to a hybridizable extent.

When the TaqMan PCR method is used, to a specimen are added the polynucleotide set (primer set) and a TaqMan probe, dNTPs (dATP, dGTP, dCTP, and dTTP), a reverse transcriptase, and a DNA polymerase (having 5' →3' exonuclease activity), and a PCR reaction is allowed to proceed while irradiating an excitation wavelength for the labeling substance.

In addition, when a primer is being extended in a PCR reaction, it is necessary that the TaqMan probe is bound to a DNA. For this reason, it is preferable that a Tm value of the TaqMan probe is higher than a Tm value of the primer. More preferably, a Tm value of the TaqMan probe is higher than a Tm value of the primer by 8 to 12°C. Thereby, under the same stringency, the TaqMan probe can bind to a DNA firmer than the primer.

The Tm value depends on a sequence and a length of the primer. As there are more GCs in a sequence of the primer, and/or as the primer is longer, the Tm value becomes higher. In addition, when a minor groove binder (MGB) which binds to a minor groove of a DNA is bound to the TaqMan probe, since a Tm value of the TaqMan probe is increased, a shorter probe can be designed without changing the same Tm value.

Fig. 2 (a) shows a schematic view of a nucleic acid for accuracy control which is a double-stranded DNA, having a first Uni sequence, a first BS sequence, a sequence for a probe, a second BS sequence and a second Uni sequence in this order from a 5' -end side. Fig. 2 (b) shows a schematic view showing with which region of the nucleic acid for accuracy control shown in Fig. 2(a), a primer and a probe hybridize.

In addition, upon quantitative real time PCR, two kinds of probes (first probe and second probe) may be used. In this case, the first labeling substance can be bound to the first probe, and the second labeling substance can be bound to the second probe. Alternatively, the first labeling substance and the second labeling substance may be bound to both ends of the first probe, respectively, and further, the first labeling substance and the second labeling substance may be bound to both ends of the second probe, respectively. Thereby, a fluorescent intensity of a reaction solution can be increased.

The aforementioned polynucleotides can be produced by the known method. For example, they can be chemically synthesized using an apparatus such as Expedite Model 8909 DNA synthesizer manufactured by ABI. Alternatively, a natural nucleic acid is cut with a restriction enzyme or the like so that a polynucleotide is constructed of the aforementioned nucleotide sequence, and this can be also used as the polynucleotide of the present embodiment.

In addition, the nucleic acid for accuracy control may contain a sequence different from the aforementioned sequences, in addition to the Uni sequence and the BS sequence. For example, in the nucleic acid for accuracy control shown in Fig. 2(a), a sequence of a few nucleotides may be contained between the first BS sequence and the sequence for a probe.

A reagent kit which is another embodiment of the present invention is a reagent kit which is used in the method for confirming conversion treatment, and contains a nucleic acid for accuracy control, and a polynucleotide for detecting a BS sequence contained in the nucleic acid for accuracy control and/or a BS sequence in which cytosine is converted into another sequence. In addition, it is preferable that the reagent kit has a polynucleotide being capable of hybridizing with a Uni sequence for calculating the aforementioned recovery rate.

### EXAMPLES

### (Example 1)

### 1. Preparation of nucleic acid for accuracy control

Invitrogen was entrusted to synthesize three kinds of polynucleotides having the following sequences.

5′-gtttatgtttttgggtgttggaag
gggccggcaagccacgaacccaccc-3′ (SEQ ID No. 6)

5'-cctctctcaaattcttatcccctcat
cctcggtggcgcgtgcggctcgggg-3' (SEQ ID No. 7)

5'-gggccggcaagccacgaacccaccc    tgtgggtgggttgtgaggtggtg
ccccgagccgcacgcgccaccgagg-3' (SEQ ID No. 8)

Using the above polynucleotides, 25 µL of a PCR reaction solution was prepared using a polynucleotide of SEQ ID No. 6 as a forward primer, a polynucleotide of SEQ ID No. 7 as a reverse primer, and a polynucleotide of SEQ ID No. 8 as a template DNA, as follows:

| | |
|---|---|
| 10 × Buffer | 2.5 µL |
| 10 mM dNTP | 0.5 µL |
| Forward primer (10 µM) | 1 µL |
| Reverse primer (10 µM) | 1 µL |
| Template DNA (0.2 µM) | 1 µL |
| Taq polymerase | 0.2 µL |
| dH₂O | 18.8 µL |

Using the above reaction solution, PCR was performed under the following conditions:
95°C for 4 minutes and 30 seconds
40 cycles of 95°C for 30 seconds, 60°C for 15 seconds, and 72°C for 15 seconds
72°C for 5 minutes

An amplification product after the PCR reaction was incorporated into a vector using a TA cloning kit (Invitrogen), and this vector was transformed into Escherichia coli (TOP 10). This Escherichia coli was cultured (LB medium, 37°C, stood still overnight), and a plasmid was purified from colonies of Escherichia coli by QIAprep Spin (Qiagen). A nucleotide sequence of a DNA incorporated into a cloning site from the purified plasmid was determined using ABI Prism 3100 (Applied Biosystems) by the BigDye terminator Cycle Sequencing method according to the attached protocol. This nucleotide sequence was as follows:

5′-gtttatgtttttggggtgttggaaggggccggcaagccacgaacccaccctg

tgggtgggttgtgaggtggtgccccgagccgcacgcgccaccgaggatgaggggataagaa

tttgagagagg-3′ (SEQ ID No. 9)

This was used as the nucleic acid for accuracy control in the following experiment. Since cytosine in a DNA synthesized by PCR is non-methylated cytosine, all cytosines in this nucleic acid for accuracy control are methylated cytosines.

First to 25^{th} sequences of the nucleic acid for accuracy control correspond to the first Uni sequence, 26^{th} to 50^{th} sequences correspond to the first BS sequence, 52^{nd} to 73^{rd} sequences correspond to the sequence for a probe, 74^{th} to 98^{th} sequences correspond to the second BS sequence, and 101^{st} to 124^{th} sequences correspond to the second Uni sequence.

### 2. Confirmation of conversion treatment

### 2. (1) Detection of methylation of nucleic acid for accuracy control

As a specimen, the MDA MB-231 cell which is a cultured cell derived from a human breast cancer was used. The human genome was extracted from the MDA MB-231 cell (1 × 10⁶ cells) using a kit manufactured by Qiagen (trade name: Blood & Cell Culture DNA mini Kit).

Purified water was dispensed into four Eppendorf tubes (tubes 1 to 4), 10 pg of the nucleic acid for accuracy control was added to the tube 1, 10 pg of the nucleic acid for accuracy control and 4 µg of the human genome were added to the tube 2, and 4 µg of the human genome was added to the tube 3. No DNA was added to the tube 4, and this was used as a negative control (NC).

To each of the tubes 1 to 4 was added 12 µl of 10N NaOH, respectively, followed by incubation at room temperature for 10 minutes. Then, 520 µL of a 3M sodium bisulfite solution was added to each tube, followed by incubation at 50°C for 16 hours (conversion treatment). A DNA contained in this bisulfite-treated solution was purified with a DNA purification kit (Qiagen QIAquik (trade name)) (DNA purification) to obtain a sample for detection. The samples for detection obtained from the tubes 1 to 4 were used as samples for detection 1 to 4, respectively.

The sample for detection 1 was dispensed into two tubes, and these tubes were designated as tubes 1a and 1b. Similarly, the samples for detection 2 to 4 were dispensed into two tubes, respectively, and they were designated as tubes 2a and 2b, tubes 3a and 3b, and tubes 4a and 4b, respectively.

To the tubes 1a, 2a, 3a and 4a was added a BS primer set (a BS-F primer and a BS-R primer) being capable of hybridizing with the nucleic acid for accuracy control when cytosine is converted into uracil, and PCR was performed.

To the tubes 1b, 2b, 3b, and 4b was added a nonBS primer set (a nonBS-F primer and a nonBS-R primer) being capable of hybridizing the nucleic acid for accuracy control when conversion does not occur, and PCR was performed.

The sequence of each primer, a PCR reaction solution composition and PCR reaction conditions are as follows:

### (Primer sequence)

BS-F primer 5'-gggttggtaagttatgaatttattt-3' (SEQ ID No. 10)
BS-R primer 5'-cctcaataacacatacaactcaaaa-3' (SEQ ID No. 11)
nonBS-F primer 5'-aagccacgaacccacc-3' (SEQ ID No. 12)
nonBS-R primer 5'-tcatcctcggtggcg-3' (SEQ ID No. 13)

### (PCR reaction solution composition)

| | |
|---|---|
| 10 × Buffer | 2.5 µL |
| 10 mM dNTP | 0.5 µL |
| BS primer set or nonBS primer set | |
| F primer (10 µM) | 1 µL |
| R primer (10 µM) | 1 µL |
| Sample for detection | 1 µL |
| Taq polymerase | 0.2 µL |
| dH₂O | 18.8 µL |

### (PCR reaction conditions)

95°C for 4 minutes and 30 seconds
30 cycles of 95°C for 30 seconds, 60°C for 15 seconds, and 72°C for 15 seconds

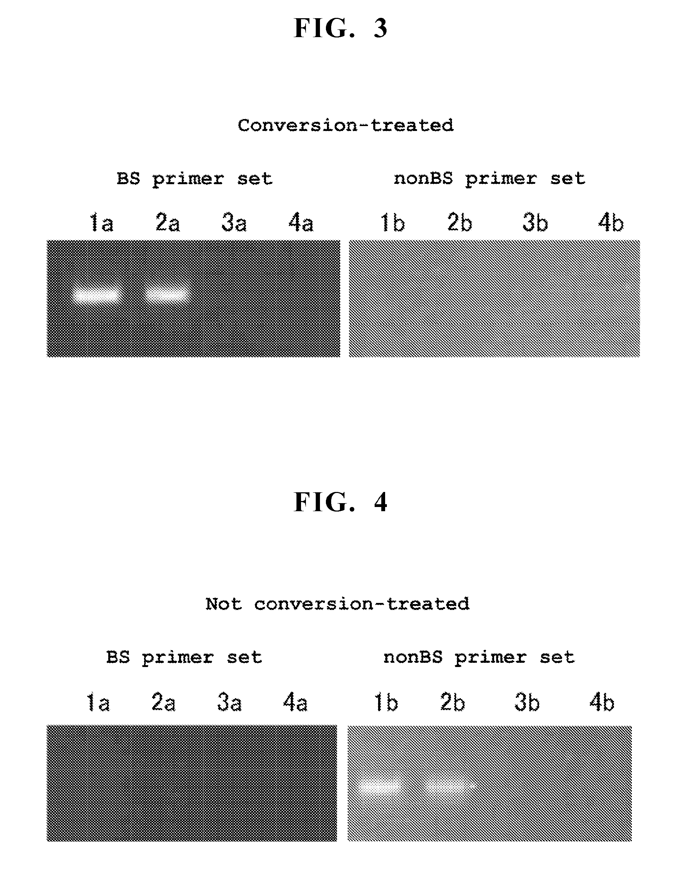

After PCR, each reaction solution was accommodated into a well of an agarose gel, and electrophoresed. After electrophoresis, the agarose gel was fluorescently stained with ethidium bromide. Fig. 3 shows a fluorescent photograph of the agarose gel.

Separately, using a sample for detection which had been prepared according to the same manner as that described above except that conversion treatment was not performed, either of the BS primer set and the nonBS primer set was added, and PCR and agarose gel electrophoresis were performed as described above. After electrophoresis, the agarose gel was fluorescently stained with ethidium bromide. Fig. 4 shows a fluorescent photograph of the agarose gel.

### 2. (2) Detection of methylation of genome

The sample for detection 1 prepared as described above was dispensed into two tubes, and these tubes were designated as tubes 1c and 1d. Similarly, the samples for detection 2 to 4 were dispensed into two tubes, respectively, and they were designated as tubes 2c and 2d, tubes 3c and 3d, and tubes 4c and 4d.

To the tubes 1c, 2c, 3c and 4c was added an Ecad primer set (an Ecad-F primer and an Ecad-R primer) being capable of hybridizing with a CpG island of an E cadherin gene (Ecad) when cytosine is converted into uracil, and PCR was performed.

To the tubes 1d, 2d, 3d, and 4d was added an ER primer set (an ER-F primer and an ER-R primer) being capable of hybridizing a CpG island of an estrogen receptor α (ERα) when cytosine is converted into uracil, and PCR was performed.

The sequence of each primer, a PCR reaction solution composition and PCR reaction conditions are as follows:

### (Primer sequence)

Ecad-F primer: 5'-ttaggttagagggttatcgcgt-3' (SEQ ID No. 14)
Ecad-R primer: 5'-taactaaaaattcacctaccgac-3' (SEQ ID No. 15)
ER-F primer: 5'-ttttgggattgtatttgttttcgtc -3' (SEQ ID No. 16)
ER-R primer: 5'-aacaaaatacaaaccgtatccccg -3' (SEQ ID No. 17)

### (PCR reaction solution composition)

| | |
|---|---|
| 10 × Buffer | 2.5 µL |
| 10 mM dNTP | 0.5 µL |
| Ecad primer set or ER primer set | |
| F primer (10 µM) | 1 µL |
| R primer (10 µM) | 1 µL |
| Sample for detection | 1 µL |
| Taq polymerase | 0.2 µL |
| dH₂O | 18.8 µL |

### (PCR reaction conditions)

95°C for 4 minutes and 30 seconds
30 cycles of 95°C for 30 seconds, 60°C for 15 seconds, and 72°C for 15 seconds

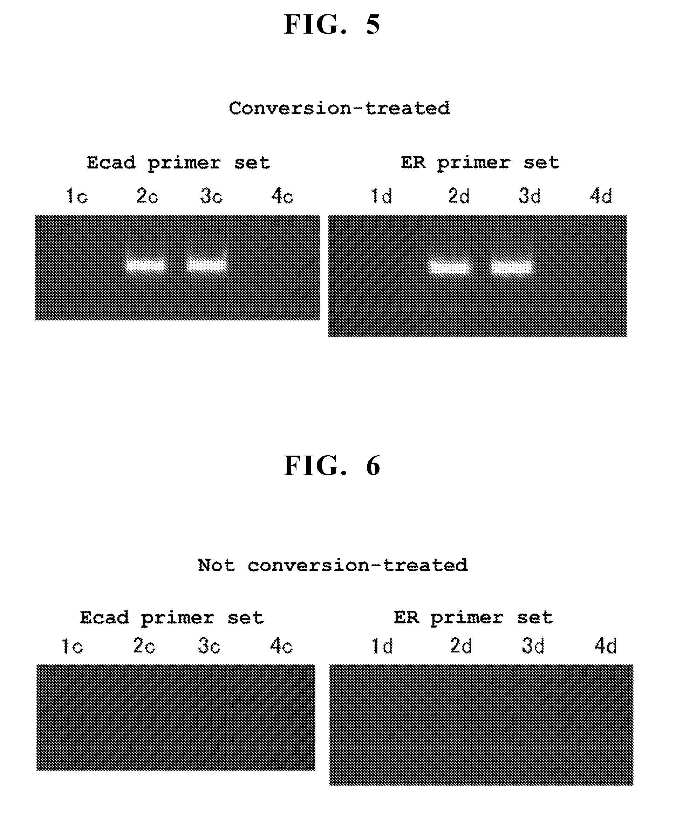

After PCR, each reaction solution was accommodated into a well of an agarose gel, and electrophoresed. After electrophoresis, the agarose gel was fluorescently stained with ethidium bromide. Fig. 5 shows a fluorescent photograph of the agarose gel.

Separately, using a sample for detection which had been prepared according to the same manner as that described above except that conversion treatment was not performed, either of the BS primer set and the nonBS primer set was added, and PCR and agarose gel electrophoresis were performed as described above. After electrophoresis, the agarose gel was fluorescently stained with ethidium bromide. Fig. 6 shows a fluorescent photograph of the agarose gel.

### 3. Results

From Fig. 3, in the tubes 1a and 2a containing the nucleic acid for accuracy control, a fluorescent band was observed when the BS primer set was used, and a fluorescent band was not observed in other tubes. From Fig. 4, in the tubes 1a and 2a containing the nucleic acid for accuracy control, a fluorescent band was confirmed when the nonBS primer set was used, and a fluorescent band was not observed in other tubes. From the forgoing, it is seen that conversion treatment on a specimen was proper.

From Fig. 5, when conversion treatment was performed, a fluorescent band was observed in the tubes 2c, 3c, 2d and 3d containing a genome. In addition, from Fig. 6, when conversion treatment was not performed, a fluorescent band was not observed in any tube. Therefore, it is seen that the CpG island of Ecad in a specimen is not methylated, and the CpG island of ERα is also not methylated. Since it was confirmed that conversion treatment was proper from the result of detection of methylation of the nucleic acid for accuracy control, the detection result of methylation of Ecad and ERα with higher reliance could be obtained.

### (Example 2)

According to the same manner as that of Example 1, a nucleic acid for accuracy control was mixed into the human genome extracted from the MDA MB-231 cell, and this was dispensed to prepare samples for detection 5 and 6.

Immediately after mixing, a mass of the nucleic acid for accuracy control was measured by TaqMan PCR. After conversion treatment and DNA purification were performed on the sample for detection 5 as in Example 1, a mass of the nucleic acid for accuracy control was measured by TaqMan PCR. After DNA purification was performed on the sample for detection 6 without conversion treatment, a mass of the nucleic acid for accuracy control was measured by TaqMan PCR.

A percentage of a mass of the nucleic acid for accuracy control after DNA purification relative to a mass of the nucleic acid for accuracy control measured immediately after mixing was calculated as a recovery rate.

TaqMan PCR was performed as follows:

The sequences of primers and a TaqMan probe, a PCR reaction solution composition and PCR reaction conditions are as follows. Here, TaqMan PCR was performed using Brilliant QPCR Master Mix (Stratagene) as a reagent, and Mx3005P Real-time PCR system (Stratagene) as an apparatus according to the attached instructions.

### (Primer sequence)

Uni-F primer: 5'-gtttatgtttttggggtgttgga-3' (SEQ ID No. 18)
Uni-R primer: 5'-cctctctcaaattcttatcccctc-3' (SEQ ID No. 19)

### (TaqMan probe sequence)

5'-tgggtgggttgtgaggtgg-3' (SEQ ID No. 20)

### (PCR reaction solution composition)

| | |
|---|---|
| 2 × Brilliant Master Mix | 12.5 µL |
| Uni-F primer (10 µM) | 1 µL |
| Uni-R primer (10 µM) | 1 µL |
| TaqMan probe (100 µM) | 0.25 µL |
| Sample for detection | 1 µL |
| Reference Dye | 0.375 µL |
| dH₂O | 8.875 µL |

### (PCR reaction conditions)

95°C for 10 minutes
40 cycles of 95°C for 30 seconds, 60°C for 15 second, and 72°C for 15 seconds

A cycle number at a time when a fluorescent intensity of the reaction solution exceeded Threshold (a value automatically set with software mounted on Mx3005P Real-time PCR system) (hereinafter, referred to as a detection cycle number) is shown in Table 1. Experiments were performed two times, respectively, and Table 1 shows an average value of detection cycle numbers of two times of experiments.

**Table 1**

| | Sample for detection 5 | Sample for detection 6 |
|---|---|---|
| Immediately after mixing step (pg) | 759.4 | 759.4 |
| After DNA purification (pg) | 0.81 | 375.6 |
| Recovery rate (%) | 0.11 | 49.46 |

A mass of the nucleic acid for accuracy control which was measured immediately after the mixing step was 759.4 pg, a mass of the nucleic acid for accuracy control which was measured after conversion treatment and DNA purification was 0.81 pg, and a mass of the nucleic acid for accuracy control which was measured after only DNA purification without conversion treatment was 375.6 pg. Therefore, the recovery rate of the nucleic acid for accuracy control of the sample for detection 5 was 0.1%, and the recovery rate of the nucleic acid for accuracy control of the sample for detection 6 was 49.5%. From the forgoing, the recovery rate of the nucleic acid for accuracy control could be measured using the sample for detection in which a specimen and the nucleic acid for accuracy control of the present Example coexisted.

The foregoing detailed description and examples have been provided by way of explanation and illustration, and are not intended to limit the scope of the appended claims. Many variations in the presently preferred embodiments will be obvious to one of ordinary skill in the art, and remain within the scope of the appended claims and their equivalents.

## Claims

1. A method for confirming conversion treatment of non-methylated cytosine, comprising steps of:
mixing a DNA taken from a living body with a nucleic acid for accuracy control, wherein the nucleic acid for accuracy control has a sequence A which is not contained in a genome of the living body and contains cytosine;
converting non-methylated cytosine in the mixture into a base other than cytosine;
detecting at least one selected from the sequence A and a sequence A' which has a sequence obtained by converting cytosine in the sequence A into the base other than cytosine; and
determining whether the conversion step has been properly performed or not based on the detection result.

2. The method according to claim 1, wherein the detection step is performed so as to detect the sequence A and the sequence A'.

3. The method according to claim 2, wherein the detection step is performed so as to detect the sequence A by using a polynucleotide being capable of hybridizing with the sequence A and the sequence A' by using a polynucleotide being capable of hybridizing with the sequence A'.

4. The method according to claim 2, wherein the sequence A contains non-methylated cytosine as the cytosine and,
when the sequence A' is detected in the detection step, the determination step is performed so as to determine that the conversion step has been properly performed.

5. The method according to claim 2, wherein the sequence A contains methylated cytosine and does not contain non-methylated cytosine as the cytosine and,
when the sequence A is detected in the detection step, the determination step is performed so as to determine that the conversion step has been properly performed.

6. The method according to claim 1, wherein the nucleic acid for accuracy control further has a sequence B which is not contained in the genome of the living body, and does not contain non-methylated cytosine.

7. The method according to claim 6, further comprising steps of:
quantitating the nucleic acid for accuracy control by using a polynucleotide being capable of hybridizing with the sequence B, after the conversion step; and
calculating a recovery rate of the DNA taken from the living body after the conversion step, wherein the recovery rate is calculated based on the quantitation result and an amount of the nucleic acid for accuracy control used in the mixing step.

8. A nucleic acid molecule comprising at least one of sequences selected from,
(a) a nucleotide sequence of SEQ ID No. 1;
(b) a nucleotide sequence in which at least one nucleotide is substituted, deleted, inserted or added in the nucleotide sequence of SEQ ID No. 1, and which is not contained in the human genome;
(c) a nucleotide sequence of SEQ ID No. 2; and
(d) a nucleotide sequence in which at least one nucleotide is substituted, deleted, inserted or added in the nucleotide sequence of SEQ ID No. 2, and which is not contained in the human genome.

9. The nucleic acid molecule according to claim 8 comprising:
the nucleotide sequence (a) or (b); and
the nucleotide sequence (c) or (d).

10. The nucleic acid molecule according to claim 8 further comprising at least one of sequences selected from,
(e) a nucleotide sequence of SEQ ID No. 3;
(f) a nucleotide sequence in which at least one nucleotide is substituted, deleted, inserted or added in the nucleotide sequence of SEQ ID No. 3, and which is not contained in the human genome;
(g) a nucleotide sequence of SEQ ID No. 4; and
(h) a nucleotide sequence in which at least one nucleotide is substituted, deleted, inserted or added in the nucleotide sequence of SEQ ID No. 4, and which is not contained in the human genome.

11. The nucleic acid molecule according to claim 10 comprising:
the nucleotide sequence (e) or (f); and
the nucleotide sequence (g) or (h).

12. A reagent kit for confirming conversion treatment of non-methylated cytosine, comprising:
a nucleic acid for accuracy control having a sequence A, wherein the sequence A is not contained in a genome of a living body and contains cytosine; and
at least one polynucleotide selected from a polynucleotide being capable of hybridizing with the sequence A and a polynucleotide being capable of hybridizing with a sequence A', wherein the sequence A' having a sequence obtained by converting cytosine in the sequence A into the base other than cytosine.

13. The reagent kit according to claim 12, wherein the nucleic acid for accuracy control has at least one of the sequences A selected from,
(a) a nucleotide sequence of SEQ ID No. 1;
(b) a nucleotide sequence in which at least one nucleotide is substituted, deleted, inserted or added in the nucleotide sequence of SEQ ID No. 1, and which is not contained in the human genome;
(c) a nucleotide sequence of SEQ ID No. 2; and
(d) a nucleotide sequence in which at least one nucleotide is substituted, deleted, inserted or added in the nucleotide sequence of SEQ ID No. 2, and which is not contained in the human genome.

14. The reagent kit according to claim 13, wherein the nucleic acid for accuracy control has the nucleotide sequence (a) or (b) and the nucleotide sequence (c) or (d) as the sequences A.

15. The reagent kit according to claim 12 further comprising a converting agent for converting non-methylated cytosine in a DNA into a base other than cytosine.

16. The reagent kit according to claim 15, wherein the converting agent is bisulfite.

17. The reagent kit according to claim 12, wherein the nucleic acid for accuracy control further has a sequence B which is not contained in the genome of the living body and does not contain non-methylated cytosine.

18. The reagent kit according to claim 17, wherein the nucleic acid for accuracy control has at least one of the sequences B selected from,
(e) a nucleotide sequence of SEQ ID No. 3;
(f) a nucleotide sequence in which at least one nucleotide is substituted, deleted, inserted or added in the nucleotide sequence of SEQ ID No. 3, and which is not contained in the human genome;
(g) a nucleotide sequence of SEQ ID No. 4; and
(h) a nucleotide sequence in which at least one nucleotide is substituted, deleted, inserted or added in the nucleotide sequence of SEQ ID No. 4, and which is not contained in the human genome.

19. The reagent kit according to claim 18, wherein the nucleic acid for accuracy control has the nucleotide sequence (e) or (f) and the nucleotide sequence (g) or (h) as the sequences B.

20. The reagent kit according to claim 18, further comprising a polynucleotide being capable of hybridizing with the sequence B.
